# EUROPEAN PATENT APPLICATION

(11) **EP 2 471 387 A1**
(43) Date of publication of application: **04.07.2012**
(21) Application number: 10197298.2
(22) Date of filing: 29.12.2010
(51) Int. Cl.: A23L 1/30, A61K 31/23, A61K 9/00, A61K 9/16

(54) **Cetyl myristate and/or cetyl palmitate suspension formulations**

(71) Applicant: Deva Holding Anonim Sirketi, Kucukcekmece/Istanbul (TR)
(72) Inventor: Haas, Philipp Daniel, 34303, ISTANBUL (TR); Firat, Omer Faruk, 34303, ISTANBUL (TR); Kandemir, Levent, 34303, ISTANBUL (TR); Koc, Fikret, 34303, ISTANBUL (TR); Sivasligil, Ramazan, 34303, ISTANBUL (TR)

(57) **Abstract**

This invention is related to a preparation method of cetyl myristate or cetyl palmitate or combination of cetyl myristate and cetyl palmitate by using dry granulation techniques wherein obtained granules having a bulk density equal or greater than 0.30 g/mL.

## Description

### Technical Field

This invention is related to a preparation method of cetyl myristate or cetyl palmitate or combination of cetyl myristate and cetyl palmitate by using dry granulation techniques wherein granules have a bulk density equal or greater than 0.30 g/mL.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to **US** US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses that cetyl myristate or cetyl palmitate or combination of cetyl palmitate and cetyl myristate are prepared by dry granulation in which granules have a bulk density equal or greater than 0.30 g/mL.

### Technical Problem

Cetyl myristate or cetyl palmitate or combination of cetyl myristate and cetyl palmitate can be used as active pharmaceutical ingredients (API) in pharmaceutical and/or dietary supplement formulations. Cetyl myristate and cetyl palmitate are used in high amounts (80 %-85 %) in pharmaceutical or dietary supplement formulations. This kind of high drug loads bring about that API(s) and excipients cannot be fitted into pharmaceutical and/or dietary dosage forms, such as capsule, which can be ingested or easily ingested by patients. High drug load also gives rise to manufacturing of huge tablets and/or capsules which are not patient compliant.

### Solution to Problem

It is invented that fitting problem for pharmaceutical and/or dietary dosage form and unproper size problem of tablets and capsules are solved by bulk density adjustment through dry granulation.

### Description of embodiments

Adjusting and determining of bulk density is so vital in the aspect of cetyl myristate or cetyl palmitate or combination of cetyl myristate and cetyl palmitate granules.

Low bulk density causes large tablet and capsule forms where API or API(s) are present at high doses in pharmaceutical formulations. Thus it will be difficult for some patients to swallow and be unacceptable to them. Additionally, if bulk density is overly reduced, poor flowing granules are obtained.

Cetyl myristate or cetyl palmitate or combination of cetyl myristate and cetyl palmitate are administered in pharmaceutical formulations in high doses (generally in total 350 mg) and drug load is high in these formulations (generally 80 %- 85 %).

If a preparation method is applied without taking into account bulk density, large tablets or capsules are obtained. It is invented that, to eliminate this problem, bulk density of granules should be higher from a critical point.

It is also invented that dry granulation method provides the aimed high bulk density.

High dose formulations of (80%-85%) cetyl myristate or cetyl palmitate or combination of cetyl myristate and cetyl palmitate need to have high bulk density (at least 0.30 g/ml).

In one aspect, this invention provides a dry granulation method for cetyl myristate or cetyl palmitate or combination of cetyl myristate and cetyl palmitate to obtain high dosage strengths involving minimal amounts of pharmaceutical excipients and having optimum sized tablets and capsules.

The present invention also provides novel granules of cetyl myristate or cetyl palmitate or combination of cetyl myristate and cetyl palmitate those provide high bulk density.

In another aspect, this invention delineates a dry granulation process to achieve target bulk density.

According to this invention granular composition of cetyl myristate or cetyl palmitate or combination of cetyl myristate and cetyl palmitate has a bulk density greater than about 0.30 g/mL.

Preferably, granular composition of cetyl myristate or cetyl palmitate or combination of cetyl myristate and cetyl palmitate has a bulk density from about 0.30 g/mL to about 1 g/mL.

More preferably, granular composition of cetyl myristate or cetyl palmitate or combination of cetyl myristate and cetyl palmitate has a bulk density from about 0.35 g/mL to about 0.60 g/mL.

Most preferably granular composition of cetyl myristate or cetyl palmitate or combination of cetyl myristate and cetyl palmitate has a bulk density from about 0.37 g/mL to about 0.47 g/mL.

It is an object of this invention to provide dry granulation, high drug content and high bulk density formulations of cetyl myristate or cetyl palmitate or combination of cetyl myristate and cetyl palmitate which result in smaller sized compressed tablet or filled capsule forms.

In accordance with this invention, final product has reasonable tablet and capsule size for patient acceptance.

Another aspect of this invention is to provide dry granulation and its method.

The dry granulations produced by the processing steps disclosed herein have excellent flow properties.

Dry granulation includes mixing the ingredients, roller compacting or slugging the mix, screening or milling, preferably lubricating, and compressing and/or filling the lubricated granules into capsules or tablets.

Dry granulation may be performed by slugging or using of compacting mills such as Chilsonator (Fitzpatrick), Roller Compactor (Vector), and the Compactor Mill (Allis-Chalmers) and the like. It is preferred that the temperature of the roller system is reduced by the connection of a cooling system, preferably with chilled water having temperature range 5 - 15°C to the rollers.

Roller compactor densifies material by passing it between two high-pressure rollers. The densified material is then milled. The milled granules may then be mixed with other excipients, such as a lubricant.

In accordance with this invention, dry granulation of cetyl myristate or cetyl palmitate or combination of cetyl myristate and cetyl palmitate are prepared by following manner : (i) roller compacting the cetyl myristate or cetyl palmitate or combination of cetyl myristate and cetyl palmitate and an excipient or mixtures of excipients to manufacture a dry granulation and (ii) mixing the granulation obtained from (i), preferably adding at least one excipient and (iii) grinding material obtained from step (ii) and (iv) tabletting or filling into the capsules material obtained from (iii).

Another aspect of this invention is to provide a slugging process for the preparation of a dry granulation of high drug load of the cetyl myristate or cetyl palmitate or combination of cetyl myristate and cetyl palmitate. The material to be tabletted is compressed to a slug, which is converted to tablets by a second compression process. The slugs are then broken up into granular particles by a grinding or milling and then recompressed into tablet or filled into capsule. According to this slugging process comprises:
(i) blending the cetyl myristate or cetyl palmitate or combination of cetyl myristate and cetyl palmitate and excipients and, (ii) slugging the blend and (iii) milling and sieving the granulation and, (iv) tabletting or filling into the capsules material obtained from step (iii).

The powder, and optionally the lubricant are compressed by using slugging or roller compaction.

The granules obtained by slugging or roller compacting can be tabletted or capsulated, for instance in hard gel capsules, such as gelatin capsules.

Yet another aspect of this invention is using of higher density excipients such as fillers, binders, disintegrants, etc. which increase the bulk density in wet granulation or direct compression. According to this, higher density excipients having bulk density equal or greater than 0.5 g/mL, preferably greater than 0.7 g/mL, should be used from 10 % to 85 % by total weight of pharmaceutical composition. It causes to obtain granules having high bulk density greater than 0.30 g/mL, preferably about 0.30 g/mL to about 1 g/mL, more preferably about 0.35 g/mL to about 0.60 g/mL,most preferably about 0.37 g/mL to about 0.47 g/mL.

Wet granulation method pursuant to this invention has following steps :

i) mixing of cetyl myristate or cetyl palmitate or combination of cetyl myristate and cetyl palmitate and an excipient or mixtures of excipients

ii) Preparation of binder solution

iii) Mixing of binder solution with powder mixture.

iv) Screening of wet mass.

v) Drying of moist granules.

vi) Screening of dry granules.

vii) Mixing of screened granules preferably with further excipients.

In accordance with this invention direct compression has following steps :
(i) Mixing of cetyl myristate or cetyl palmitate or combination of cetyl myristate and cetyl palmitate and an excipient or mixtures of excipients

(ii)Compressing the tablet from mass.

Direct compression agents are, but not limited to, pregelatinised starches, polyvinylpyrrolidone, methylcellulose, microcrystalline cellulose, sucrose,lactose, dextrose, sorbitol, mannitol, lactitol,xylitol, modified calcium salt, granulated corn starch, modified rice starch, compressible sugars such as Destab™, dextrates such as Emdex ® dicalcium phosphate, hydroxypropylcellulose , methylcellulose , hydroxypropylmethylcellulose, polyethylene glycol, amylose,anhydrous calcium hydrogen phosphate, calcium sulphate,tribasic calcium phosphate, dibasic calcium phosphate,low-crystallinity powdered cellulose,silicified microcrystalline cellulose,chitin,chitosan hydrochloride,copovidone,croscarmellose sodium,dextrose,anhydrous lactose,anhydrous alpha lactose,anhydrous beta lactose,agglomerated lactose, spray-dried lactose, maltodextrin, mixtures thereof and the like.

Disintegrants are, but not limited to, alginic acid, chitosan, pectins, cation exchange resins, magnesium silicates, aluminium silicates,mixtures thereof and the like.

Lubricants/Glidants are, but not limited to, magnesium stearate,calcium stearate,hydrogenated castor oil,glyceryl behenate,glyceryl monostearate,glyceryl palmitostearate,leucine,mineral oil, light mineral oil, myristic acid,palmitic acid,polyethylene glycol,potassium benzoate,sodium benzoate,sodium lauryl sulfate,sodium stearyl fumarate,stearic acid,talc, hydrogenated vegetable oil,zinc stearate, magnesium lauryl sulphate,sodium stearyl fumarate, polyethylene glycol, stearic acid, colloidal silicon dioxide or mixtures thereof and the like. Preferred lubricant is magnesium stearate. Two different kinds of lubricants are preferred to be benefited from different proporties of lubrication.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

### Example 1

**Table 1**

| **INGREDIENTS** | **mg** |
|---|---|
| **Composition of Granules Obtained by Roller Compacting** | |
| Cetyl Myristate/API | 333.3 |
| Cetyl Palmitate/API | 16.7 |
| Microcrystalline Cellulose (Cellets 200) | 20.0 |
| Zinc Stearate | 5 |
| Lactose Anhydrous | 25 |
| Mannitol | 20 |
| **Total Granules Weight** | **420.0** |

333.3 mg cetyl myristate, 16.7 mg cetyl palmitate, 20 mg Cellets 200, 5 mg zinc stearate, 25 mg lactose anhydrous, 20 mg mannitol are blended, roller compacted, milled using a Fitzmill .

### Example 2

**Table 2**

| **INGREDIENTS** | **mg** |
|---|---|
| **Composition of Granules Obtained by Wet Granulation** | |
| Cetyl Myristate/API | 333.3 |
| Cetyl Palmitate/API | 16.7 |
| Lactose Monohydrate (Pharmatose 60M) | 22.0 |
| Sodium Lauryl Sulfate | 3 |
| Croscarmellose Sodium | 30 |
| Sorbitol (Sorbitab SD 250) | 15 |
| **Total Granules Weight** | **420.0** |

Wet granulation method pursuant to this invention has following steps :

i) mixing of cetyl myristate or cetyl palmitate or combination of cetyl myristate and cetyl palmitate and croscarmellose sodium and Sorbitab SD 250

ii) Preparing of Pharmatose 60M solution

iii) Mixing of binder solution with powder mixture obtained from (i).

iv) Screening of wet mass.

v) Drying of moist granules.

vi) Screening of dry granules.

vii) Mixing of screened granules with Sodium Lauryl Sulfate.

### Example 3

**Table 3**

| **INGREDIENTS/FUNCTI ONS** | **mg** |
|---|---|
| **Composition Obtained by Direct Compression** | |
| Cetyl Myristate/API | 333.3 |
| Cetyl Palmitate/API | 16.7 |
| Lactose Monohydrate (Pharmatose 60M) | 27 |
| Sodium Starch Glycolate (Primojel) | 10 |
| Sodium Lauryl Sulfate | 3 |
| Dextrose | 30 |
| Sorbitol (Sorbitab SD 250) | 19 |
| **Total Granules Weight** | **439.0** |

a. Dextrose, cetyl myristate, cetyl palmitate and lactose monohydrate are installed into a container and mixed,

b. Sodium Starch Glycolate and Sodium Lauryl Sulfate are installed and mixed with the mass obtained from step a,

c. Sorbitol and the mass obtained from step b are mixed,

d.The final mixture is compressed in a tablet

## Claims

1. Granules of cetyl myristate or cetyl palmitate or combination of cetyl myristate and cetyl palmitate **characterized in that** granules have a bulk density equal or greater than 0.30 g/mL.

2. Bulk density of granulated composition of cetyl myristate or cetyl palmitate or combination of cetyl myristate and cetyl palmitate, as claimed in claim 1, is from 0.30 g/mL to about 1 g/mL.

3. Bulk density of granulated composition of cetyl myristate or cetyl palmitate or combination of cetyl myristate and cetyl palmitate, as claimed in claim 2, is from 0.35 g/mL to about 0.60 g/mL.

4. Bulk density of granulated composition of cetyl myristate or cetyl palmitate or combination of cetyl myristate and cetyl palmitate, as claimed in claim 3, is from about 0.37 g/mL to about 0.47 g/mL.

5. According to preceding claims, granules having high bulk density are prepared by dry granulation.

6. According to claim 5, dry-granulation tablets or granules comprising cetyl myristate or cetyl palmitate or combination of cetyl myristate and cetyl palmitate obtained by roller compaction or by slugging.

7. Roller compaction as claimed in claim 6 is performed for dry granulation by following manner : (i) roller compacting the cetyl myristate or cetyl palmitate or combination of cetyl myristate and cetyl palmitate and an excipient or mixtures of excipients to manufacture a dry granulation and (ii) mixing the granulation obtained from (i), preferably with at least one excipient and (iii) grinding material obtained from step (ii) and (iv) tabletting or filling into the capsules material obtained from (iii).

8. Slugging as claimed in claim 6 is performed for dry granulation by following manner :(i) blending the cetyl myristate or cetyl palmitate or combination of cetyl myristate and cetyl palmitate and excipients and, (ii) slugging the blend and (iii) milling and sieving the granulation and, (iv) tabletting or filling into the capsules material obtained from step (iii).

9. Granules of cetyl myristate or cetyl palmitate or combination of cetyl myristate and cetyl palmitate **characterized in that** higher density excipients have a bulk density equal or greater than 0.5 g/mL, preferably equal or greater than 0.7 g/mL, are used from 10 % to 85 % by weight of pharmaceutical or dietary supplement composition.

10. According to claim 9, granules of cetyl myristate or cetyl palmitate or combination of cetyl myristate and cetyl palmitate are prepared by wet granulation or direct compression.

11. Wet granulation as claimed in claim 10 comprises: i) mixing of cetyl myristate or cetyl palmitate or combination of cetyl myristate and cetyl palmitate and an excipient or mixtures of excipients, ii) Preparation of binder solution, iii) Mixing of binder solution with powder mixture, iv) Screening of wet mass, v) Drying of moist granules, vi) Screening of dry granules, vii) Mixing of screened granules with further excipients.

12. Direct compression as claimed in claim 10 comprises: (i) mixing of cetyl myristate or cetyl palmitate or combination of cetyl myristate and cetyl palmitate and an excipient or mixtures of excipients and (ii) compressing the tablet from mass.
